(19) Europäisches Patentamt
European Patent Office
Office européen des brevets

(11) **EP 3 995 566 A1**

(12) **EUROPEAN PATENT APPLICATION**
published in accordance with Art. 153(4) EPC

(43) Date of publication:
**11.05.2022 Bulletin 2022/19**

(21) Application number: **20837127.8**

(22) Date of filing: **06.07.2020**

(51) International Patent Classification (IPC):
***C12M 3/00*** (2006.01)   ***C12N 5/02*** (2006.01)

(52) Cooperative Patent Classification (CPC):
**C12M 3/00**

(86) International application number:
**PCT/JP2020/026399**

(87) International publication number:
**WO 2021/006242 (14.01.2021 Gazette 2021/02)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.07.2019 JP 2019125709**

(71) Applicant: **Kinki University
Higashi-Osaka-shi
Osaka 577-8502 (JP)**

(72) Inventors:
• **KUSUNOKI Masanobu
  Kinokawa-shi, Wakayama 649-6493 (JP)**
• **TAKEUCHI Hiroki
  Tsu-shi, Mie 514-2303 (JP)**
• **TOGO Hidetaka
  Kyoto-shi, Kyoto 610-1121 (JP)**

(74) Representative: **Gill Jennings & Every LLP
The Broadgate Tower
20 Primrose Street
London EC2A 2ES (GB)**

(54) **CELL CULTURE SCAFFOLD, PRODUCTION METHOD AND SCAFFOLD PRODUCTION KIT THEREFOR, AND CELL CULTURE CREATION METHOD**

(57)     An object is to provide a cell culture scaffold which makes it possible to recover a cell culture product without being destroyed after cell culturing and a method for producing a cell culture product.

The cell culture scaffold according to the present invention includes a substrate having a water-repellent surface and a cell adhesion molecule formed on the water-repellent surface of the substrate. Further, the method for producing a cell culture product according to the present invention includes a step of culturing, using the cell culture scaffold, cells on the surface of the cell adhesion molecule. The cell culture scaffold according to the present invention makes it possible to recover the cells in a spheroid state without causing a damage.

FIG. 6

EP 3 995 566 A1

**Description**

Field

The present invention relates to a cell culture scaffold for culturing a cell and a method for producing a cell culture product (particularly a three-dimensional cell aggregate) using the cell culture scaffold.

Background

For detaching a cell culture product from a culture scaffold after cell culturing, a method using an enzyme is known. However, it is difficult to recover the cell culture product without breaking intercellular junction in this method.
Furthermore, a method is known that includes using a temperature-responsive polymer in a cell culture scaffold and decomposing the temperature-responsive polymer via heat treatment to detach and recover a cell culture product from the culture scaffold (for example, see Patent Literature 1, etc.).
In regenerative medicine, it is highly desirable that cultured cells be recovered as spheroids. Culturing cells into spheroids is proposed by Non-patent Literature 1 and the like. In Non-patent Literature 1, hydroxyapatite is used to form a dot pattern on a polytetrafluoroethylene (PTFE) surface and cells are cultured on such a surface to form spheroids.
Furthermore, as a cell carrier, a substrate in which an extracellular matrix, composed of a material such as a protein or a peptide, is coated on a hydrophobic substrate is known (Patent Literature 2).

Citation List

Patent Literature

     Patent Literature 1: Japanese Patent Application Laid-Open No. 2013-099282
     Patent Literature 1: Japanese Patent Application Laid-Open No. 2016-214210

Non-Patent Literature

Non-Patent Literature 1: Tamura et. al., "Studies on apatite dot production methods for obtaining cell/spheroid arrays," Japanese Society for Medical and Biological Engineering Symposium 2013 (proceedings, p141), presented on September 20, 2013.

Summary

Technical Problem

In the method in Patent Literature 1, culturing and detaching processes need to be performed under temperatures controlled to be within a narrow margin, which is difficult to achieve. In addition, the size control is not easy to perform.
Non-patent Literature 1 describes that cells are cultured into a spheroid-like state. However, there is no description of detaching the cells from the substrate. Patent Literature 2 describes that the extracellular matrix is coated on the hydrophobic substrate. However, this technique is not originally intended to form spheroids.
The following problems occur when conventional cells are cultured into three-dimensionally aggregated spheroids and recovered.

     (1) Even if spheroids are formed, detachment of the spheroids from the substrate without causing damage to the spheroids is difficult.
     (2) It is difficult to produce free-floating spheroids that are uniform in size.
     (3) It is difficult to produce spheroids in which all constituting cells have undergone differentiation by induction.
     (4) It is difficult to produce spheroids in which all cells constituting the spheroids have proper cell polarity.

The methods described in the above-mentioned prior literatures fail to solve these problems and thus it has been difficult to recover spheroids of the cells.
Thus, an object of the present invention is to provide a cell culture scaffold which makes it possible to recover a cell culture product without being destroyed after cell culturing and also provide a method for producing a cell culture product using the cell culture scaffold.

Solution to Problem

The present invention includes the following configuration to solve the problems described above.

That is, a cell culture scaffold according to the present invention includes a substrate having a water-repellent surface made of a fluororesin and a cell adhesion molecule formed on the water-repellent surface of the substrate.

Further, a method for producing a cell culture product according to the present invention includes a step of culturing, using the cell culture scaffold described above, cells on a surface of the cell adhesion molecule.

Advantageous Effects of Invention

When cells are cultured using the cell culture scaffold according to the present invention, the boundary faces between the water-repellent surface made of a fluororesin and the cell adhesion molecule are firmly adhered to each other for a predetermined period of time after the start of cell culturing. However, the adhesion between the boundary faces is reduced with further passage of a predetermined period of time. After the cells are cultured to a certain stage, such a temporal change in the adhesion can be used to detach the cell culture product from the cell culture scaffold without destroying the cell culture product. Furthermore, when the cell adhesion molecules are formed in a pattern, it becomes possible to recover the cell culture products as three-dimensional cell aggregates in which the cell culture products are three-dimensionally aggregated. Multiple three-dimensional cell aggregates of about equal size can be recovered. Furthermore, as an advantage of the present invention, any difficulty in temperature control that is necessary, for example, when a temperature-responsive polymer is used, is eliminated. Furthermore, the size control can be easily performed.

As a result, it is expected that the three-dimensional spheroids can be formed as follows. Induction factors are added to two-dimensionally cultured stem cells to induce cell polarity. Then, while maintaining the cell polarity, the cells are detached from the substrate using an operation such as pipetting, which causes little damage to the cells, at an appropriate timing. This makes it possible to solve the above conventional problems that have arisen when obtaining the spheroids.

Brief Description of Drawings

Fig. 1 is a schematic view illustrating a procedure for production of a mold in a stamp method.
Fig. 2 is a schematic view illustrating a procedure for production of a micro-stamp in the stamp method.
Fig. 3 is a schematic view illustrating a procedure for transfer of cell adhesion molecules using a micro-stamp.
Fig. 4 is a schematic view illustrating a procedure for pattern formation of the cell adhesion molecules by a sealing method.
Fig. 5 is a diagram exemplifying other forms of a cell culture kit for forming spheroids.
Fig. 6 is a schematic view illustrating a state of cell culturing in which a cell culture scaffold according to the present invention is used.
Fig. 7 is a plan view of photomasks used in Examples.
Fig. 8 shows photographs of cell culture products before detaching in Examples 1 to 4.
Fig. 9 shows photographs of cell culture products after detaching in Examples 1 to 4.
Fig. 10 shows photographs of the cell culture products after culturing and detaching in Examples 5 to 9.
Fig. 11 shows photographs of the cell culture products after culturing and detaching in Examples 10 to 13.
Fig. 12 shows photographs of the cell culture products after culturing and detaching in Examples 14 to 18.
Fig. 13 shows photographs of the cell culture products after culturing and detaching in Examples 19 and 20.
Fig. 14 shows actual photographs of a pattern of the cell adhesion molecules formed by the sealing method.
Fig. 15 shows a photograph of a spheroid produced using the cell culture scaffold produced by the sealing method.

Description of Embodiments

Hereinafter, the cell culture scaffold and the method for producing a cell culture product according to the present invention will be described in detail. However, the scope of the present invention is not restricted by the following description, and those other than the following examples can also be appropriately modified and implemented to an extent in which a gist of the present invention is not impaired.

[Substrate]

A substrate of the present invention has a water-repellent surface made of a fluororesin.

As an example of the substrate having the water-repellent surface, the one in which the substrate such as a petri dish, a well, a plate, a multi-plate, or a flask is made of a material other than the fluororesin and a fluororesin layer is coated on the surface of the substrate can be used.

As an example of the substrate made of a material other than the fluororesin, an existing substrate such as the one made of plastic such as polystyrene or the one made of glass can be used. The substrate from which the fluororesin layer is not easily peeled off is appropriately selected.

The method for forming the fluororesin layer on the substrate is not particularly limited, and the fluororesin layer can be formed by coating a fluororesin layer on the substrate by a known coating method such as spin coating, dip coating, or spray coating, and heating it for curing, for example.

Furthermore, unlike the above, the fluororesin may be used as a material of the substrate as exemplified by a petri dish made of the fluororesin. Such a substrate is also included in the "substrate having the water-repellent surface made of the fluororesin" of the present invention.

As the fluororesin, a known fluororesin can be used. Preferable examples thereof include polytetrafluoroethylene, polychlorotrifluoroethylene, poly(vinylidene fluoride), poly(vinyl fluoride), perfluoroalkoxy fluororesin, an ethylene tetrafluoride-propylene hexafluoride copolymer, an ethylene-tetrafluoroethylene copolymer, and an ethylene-chlorotrifluoroethylene copolymer.

[Cell adhesion molecule]

Examples of the cell adhesion molecule include extracellular matrices such as collagen, vitronectin, fibronectin, laminin, elastin, heparan sulfate, and proteoglycan, molecules prepared by fragmenting cell adhesion sites of these extracellular matrices, and cationic polymers such as polylysine, polyethylenimine, and poly(allylamine hydrochloride).

Such a cell adhesion molecule itself is conventionally known and can be appropriately selected according to the type and the like of the cells to be cultured.

[Cell culture scaffold]

The cell culture scaffold of the present invention includes the substrate having the water-repellent surface described above and the cell adhesion molecule formed on the water-repellent surface of the substrate described above.

The cell adhesion molecule can be formed on the water-repellent surface of the substrate by a known coating method such as spin coating, dip coating, or spray coating.

Furthermore, instead of performing a simple coating, the cell adhesion molecules may be formed in a pattern on the water-repellent surface of the substrate.

Here, the term "in a pattern" will be described. A region where the cell adhesion molecules are continuously formed on the substrate having the water-repellent surface is referred to as a "cell adhesion molecule region." The cell adhesion molecule region includes an edge that serves as a boundary with the substrate. That is, the edge is a border between the cell adhesion molecule region and the substrate surface.

The term "in a pattern" refers to a state in which at least one or more cell adhesion molecule regions are formed on the substrate preferably at a plurality of locations. Each cell adhesion molecule region does not necessarily have the same shape and may vary in size. Note that the cell adhesion molecule region is also simply referred to as the "cell adhesion molecule."

When the cell adhesion molecules are formed in a pattern, the culture cells can be recovered as a three-dimensional cell aggregate.

The term "three-dimensional cell aggregate" described herein refers to cells that are three-dimensionally aggregated. A three-dimensional cell colony is referred to as a spheroid. Further, a cell aggregate formed of multiple kinds of cells that are specific to an organ is referred to as an organoid.

Unlike a two-dimensional cell culture product, such a three-dimensional cell aggregate can exhibit a function as a tissue, leading to the expectation that this aggregate can be used in various kinds of methods such as one for confirming an effect of a drug without using a human body. However, it has been conventionally difficult to recover the three-dimensional cell aggregate without destroying it.

Thus, the present invention is extremely effective as a method for recovering the three-dimensional cell aggregate without destroying it as described above. Furthermore, as an advantage of the present invention, any difficulty in temperature control that is necessary, for example, when a temperature-responsive polymer is used, is eliminated. Furthermore, the size control can be easily performed.

For example, when the cell adhesion molecules are formed in a pattern having a dot-like shape (substantially circular shape), it becomes possible to form the three-dimensional cell aggregate in a size corresponding to that of each dot.

In this case, the size of the dot (cell adhesion molecule region) can be set to, for example, 100 to 1,000 $\mu$m in

diameter. Provided that the size of the cells to be cultured is about 5 to 25 $\mu$m, when the cell adhesion molecule region is too small, the resulting spheroid is small and insufficient as tissue cells. On the other hand, when the cell adhesion molecule region is too large, internal cells undergo necrosis.

Note that, in the present invention, needless to say, the shape is not limited to the above-mentioned dot-like shape and various pattern shapes can be adopted in accordance with the intended purposes.

The pattern formation of the cell adhesion molecules is not particularly limited. However, examples thereof include a method using an ink jet printer, a stamp method, and a sealing method. The stamp method allows the size control in the order of micrometers and is thus preferable.

The sealing method makes it possible to easily and accurately form a pattern of the cell adhesion molecules without requiring a skillful technique in the pattern formation of the cell adhesion molecules. This sealing method uses a sealing material which includes through-holes having a shape of the pattern of the cell adhesion molecules (size and thickness) and is previously applied to the substrate.

The pattern formation of the cell adhesion molecules can also be performed by sticking the sealing material, including through-holes having a shape of the pattern of the cell adhesion molecules, to the substrate, applying the cell adhesion molecules to the sealing material, and peeling off the sealing material.

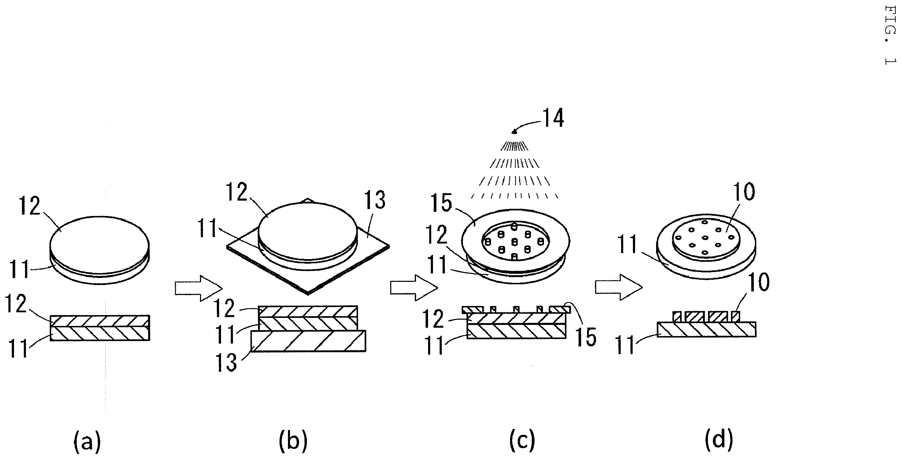

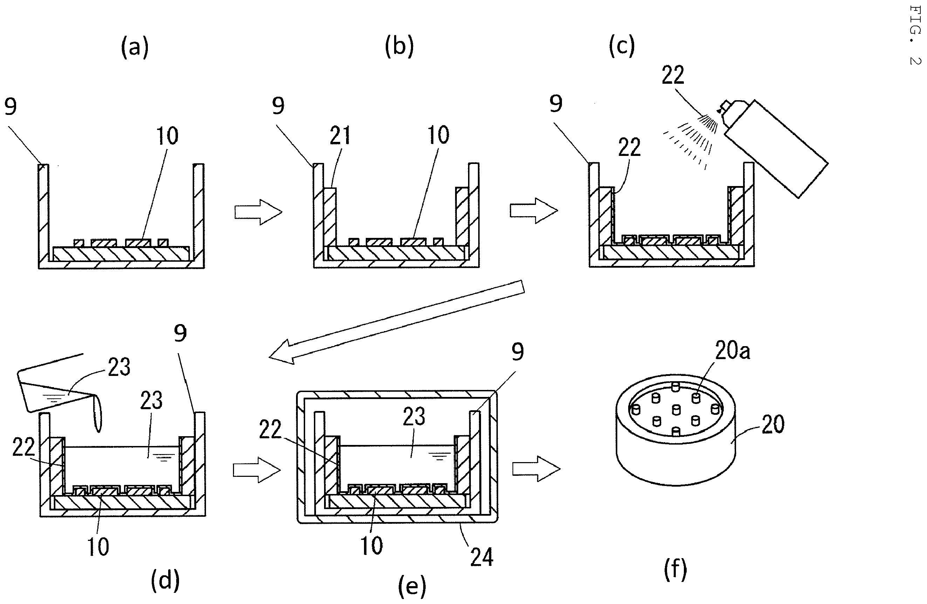

A procedure of the stamp method will be described below with reference to Figs. 1 and 2.

Fig. 1 is a diagram illustrating a procedure for production of a mold.

Initially, a resist 12 is applied on a silicon substrate 11 (Fig. 1(a)). Subsequently, the resist 12 is subjected to heat curing using a heater 13 (Fig. 1(b)) and then exposed to light through a photomask 15 using a light source 14 to form a pattern (Fig. 1(c)). Lastly, development and cleaning are performed to obtain a mold 10 (Fig. 1(d)).

Fig. 2 is a diagram illustrating a procedure for production of a micro-stamp using the mold described above.

Initially, the mold 10 is placed inside an outer container 9 (Fig. 2(a)), and, in a state in which a rubber tube 21 is disposed (Fig. 2(b)), the mold 10 described above is subjected to a release treatment using a release agent 22 (Fig. 2(c)). Subsequently, a stamp material 23 such as polydimethylsiloxane (PDMS) is poured into the mold 10 (Fig. 2(d)). Subsequently, heat curing is performed in a constant-temperature dryer 24 (Fig. 2(e)) to obtain a micro-stamp 20 (Fig. 2(f)). The micro-stamp 20 includes projection portions 20a.

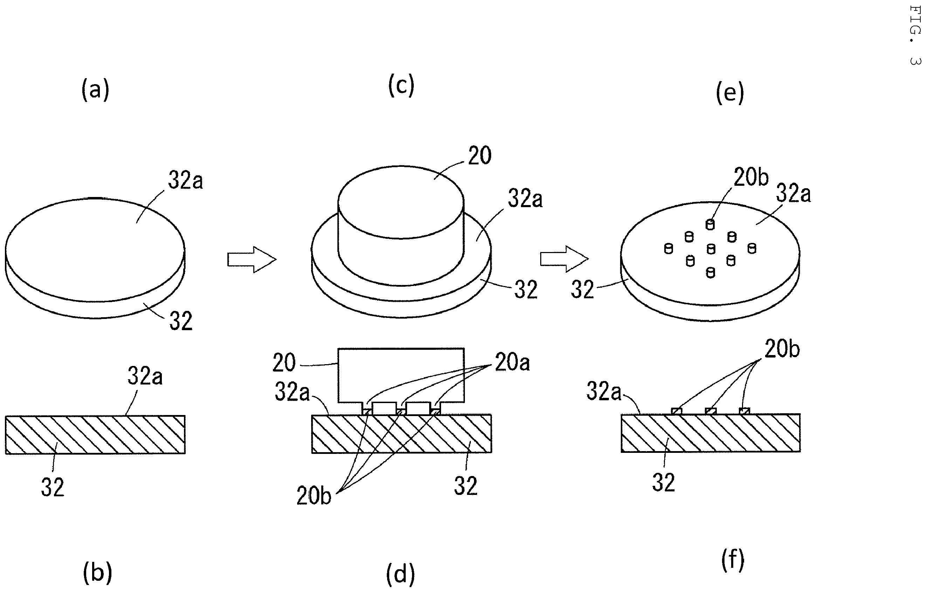

Next, Fig. 3 shows a procedure for transfer of the cell adhesion molecules using the micro-stamp described above. Fig. 3(a), Fig. 3(c), and Fig. 3(e) show a flow of the procedure, while Fig. 3(b), Fig. 3(d), and Fig. 3(f) show side views of Fig. 3(a), Fig. 3(c), and Fig. 3(e), respectively.

Referring to Fig. 3, first, a substrate 32 in which a surface 32a is subjected to a water-repelling treatment is prepared (Fig. 3(a) and Fig. 3(b)), and cell adhesion molecules 20b are applied on the surface of the projection portions 20a of the micro-stamp 20 described above. Second, the projection portions 20a of the micro-stamp 20 are made to tightly adhere to the water-repellent surface 32a of the substrate 32 (Fig. 3(c) and Fig. 3(d)). This allows the pattern of the projection portions 20a of the micro-stamp 20, exhibiting the same pattern as that of the cell adhesion molecules 20b (Fig. 3(e) and Fig. 3(f)), to be transferred to the water-repellent surface 32a of the substrate 32.

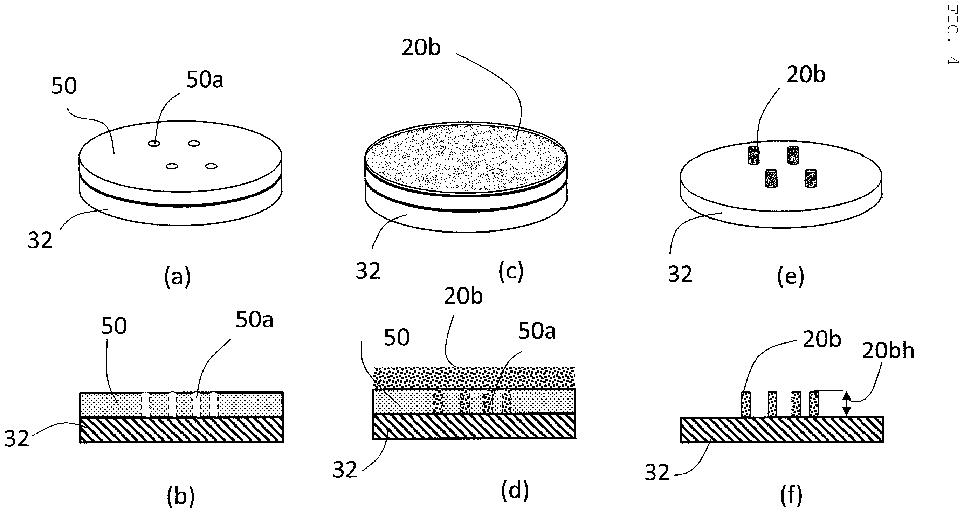

Next, the sealing method will be described. Fig. 4 shows a procedure for formation of the pattern of the cell adhesion molecules by the sealing method. Fig. 4(a), Fig. 4(c), and Fig. 4(e) show a flow of the procedure, while Fig. 4(b), Fig. 4(d), and Fig. 4(f) show side views of Fig. 4(a), Fig. 4(c), and Fig. 4(e), respectively.

A sealing material 50 in which holes 50a are formed are closely disposed on the substrate 32 (Fig. 4(a)). The sealing material 50 being closely disposed on the substrate 32 refers to a state in which there is no air inclusion between the sealing material 50 and the substrate 32. Note that "no air inclusion" refers to a state in which air inclusion that would affect the pattern formation of the cell adhesion molecules is not observed. For example, bubbles sufficiently small with respect to the film thickness of the sealing material 50 may be permitted.

A method of "closely disposing" is not particularly limited. A method in which a material of the sealing material 50 is applied on the entire surface of the substrate 32 and subsequently the holes 50 are formed may be used. Alternatively, the sealing material 50 in which the holes 50a are formed in advance may be adhered to the substrate 32.

Next, the cell adhesion molecules 20b are disposed on the top of the sealing material 50 (Fig. 4(c)). A method for the disposition of the cell adhesion molecules 20b is not particularly limited. Coating, dipping, spraying, or the like can be suitably used. Subsequently, by removal of the cell adhesion molecules 20b on the sealing material 50 and removal afterwards of the sealing material 50, the pattern of the cell adhesion molecules 20b can be obtained (Fig. 4(e)).

In the sealing method, the thickness 20bh of the final cell adhesion molecules 20b (cell adhesion molecules regions) can be easily controlled by controlling the thickness of the sealing material 50.

The sealing material 50 is required to have a characteristic in which components of the sealing material 50 on the substrate 32 do not remain when the sealing material 50 is peeled off from the substrate 32. The remnants on the substrate 32 may contaminate the cells to be cultured.

Furthermore, the sealing material 50 is required to have a characteristic of not causing deterioration of the substrate 32 itself after the sealing material 50 is peeled off from the substrate 32. In addition, the sealing material 50 is required to have a characteristic of being easily peeled off from the substrate 32 without simultaneously causing the fixed cell adhesion molecules 20b to be peeled off from the substrate 32.

The material of the sealing material 50 is not particularly limited as long as such characteristics can be achieved. A resin material such as a thermosetting resin, a thermoplastic resin, or a photosetting resin, a peptide material represented by gelatin or the like, a paper, clay (a mixture of an inorganic substance and a resin), or the like can be suitably used.

Note that closely disposing the sealing material 50 on the substrate 32 by adhesion requires a skillful technique. Thus, a cell culture kit, in which the sealing material 50 with the holes 50a being formed therein is formed on the substrate 32 in advance, is effective. A cell adhesion molecule 20b of the user's preference is used. Such a cell culture kit can be also referred to as a cell culture kit for forming spheroids in which the holes 50a are formed in the sealing material 50 and the sealing material 50 is closely disposed on the substrate 32, which includes the water-repellent surface 32a.

Furthermore, needless to say, the cell culture kit may have a configuration in which the cell adhesion molecules 20b are disposed inside the holes 50a. Such a cell culture kit can be referred to as a cell culture kit for forming spheroids in which the sealing material 50 with the holes 50a being formed therein is formed on the substrate 32, and the cell adhesion molecules 20b are disposed in the holes 50a.

Further, it is desirable that the cell culture kit for forming spheroids have holes 50a formed in the sealing material 50 that are similar in shape but variable in size. Regarding the cell culture scaffold according to the present invention, the final state of the spheroids of the cultured cells may vary depending on the size of the cell adhesion molecule regions. Thus, when the sealing material 50, which the holes 50a of variable size are formed in, is disposed on the substrate 32, determining the necessary size for culturing unfamiliar cells becomes easy, the size referring to that of the cell adhesion molecule region to be formed.

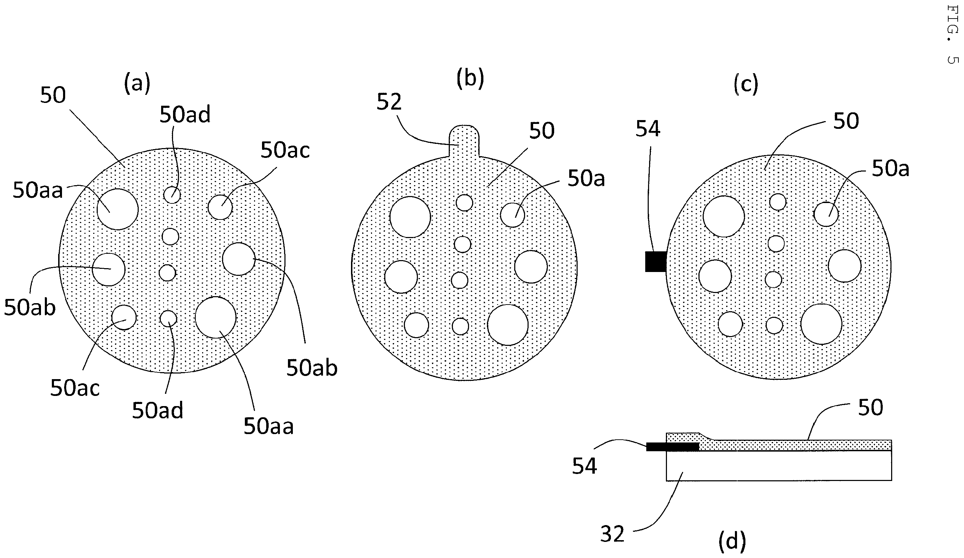

Fig. 5(a) shows an example of the cell culture kit for forming spheroids in which the holes 50a different in size are formed. The substrate 32 is behind the sealing material 50. Holes 50aa, holes 50ab, holes 50ac, and holes 50ad are the holes 50a made smaller in this order. Needless to say, variations in arrangement and size of the holes 50a are not limited to this example.

Furthermore, a projection portion or a tab to be grasped at the time of peeling may be disposed in an end portion of the sealing material 50 for easy peeling. Fig. 5(b) shows the cell culture kit for forming spheroids in which a projection portion 52 is formed. The substrate 32 is behind the sealing material 50. The projection portion 52 is a part projecting from the sealing material 50 in the same plane.

Further, Fig. 5(c) shows the cell culture kit for forming spheroids in which a tab 54 is disposed. Fig. 5(d) is a side view of the cell culture kit for forming spheroids in Fig. 5(c).

The tab 54 is a film inserted between the substrate 32 and the sealing material 50. When the sealing material 50 is peeled off, this tab is held and pulled by tweezers or the like, so that the sealing material 50 can be peeled off from the substrate 32.

[Cell culturing]

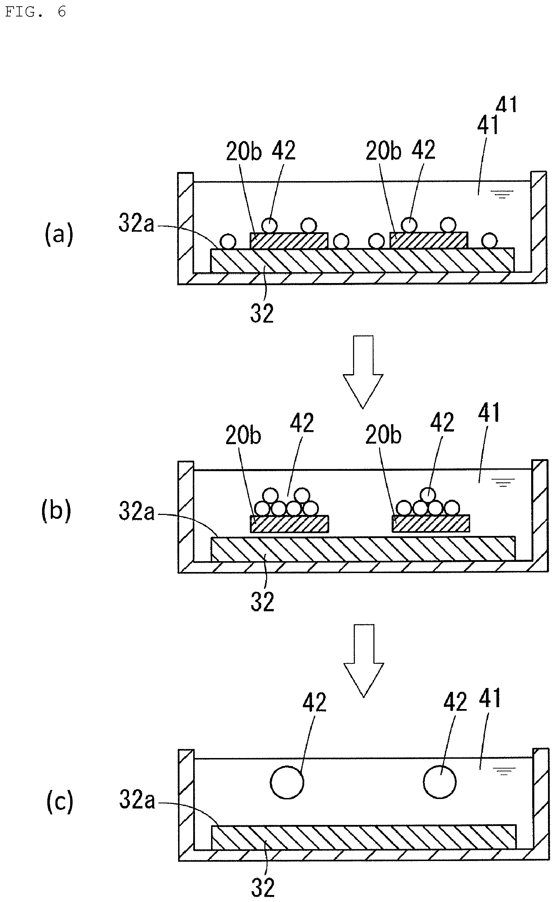

Cell culturing will be described below with reference to Fig. 6.

When cell culturing is performed in a culture liquid 41 using the cell culture scaffold of the present invention, as shown in Fig. 6, cells proliferate on the surface of the cell adhesion molecules 20b, which are formed in a pattern on the water-repellent surface 32a of the substrate 32.

Cell culturing may be performed by a conventional method with the exception that the cell culture scaffold according to the present invention is used.

At the initiation stage of the cell culturing, the boundary faces of the water-repellent surface 32a and the cell adhesion molecules 20b maintain adhesion and are not easily separated from each other. However, the adhesion of the boundary faces of the water-repellent surface 32a and the cell adhesion molecules 20b is reduced with passage of time.

As the adhesive force is reduced, the cell adhesion molecules 20b are spontaneously peeled off. Alternatively, the cell adhesion molecules 20b can be peeled off by applying stimulation, using a technique such pipetting, to the boundary faces where the adhesive force is reduced.

When the cell adhesion molecules 20b are peeled off, cell culture products 42 are detached in the culture liquid 41. Thus, by recovering the detached cell culture products 42 in the culture liquid 41, the culture products 42 can be recovered without being destroyed.

In this case, as shown in Fig. 6, the cell adhesion molecules 20b are detached together with the culture products 42 while being surrounded by them.

Stem cells are cultured on the cell adhesion molecules 20b and induction factors are added before the cell adhesion molecules 20b are peeled off from the substrate 32, so that cells differentiated from the stem cells can be recovered as spheroids.

It is basically assumed that cells applicable to the present invention are adherent cells.

Example thereof include an epithelial-like cell, a mesenchymal cell, and a fibroblast-like cell, without being particularly limited thereto. Furthermore, a pluripotent stem cell such as an ES cell or an iPS cell, a tissue-specific stem cell, a progenitor cell, and the like are also included in the applicable cells.

Examples

Hereinafter, the present invention will be described in detail by way of Examples. However, the present invention is not limited to these Examples.

[Preparation of cell adhesion molecules and culture cells]

The cell adhesion molecules and the culture cells used in each Example will be described below.

<Cell adhesion molecules>

(1) Fibronectin (5 $\mu$g/cm$^2$): fibronectin solution, from human plasma (063-05591 / FUJIFILM Wako Pure Chemical Corp.)
(2) Vitronectin (1 $\mu$g/cm$^2$): rhVTN (220-02041 / FUJIFILM Wako Pure Chemical Corp.)
(3) Matrigel (250-340 $\mu$g/ml): Corning Matrigel Basement Membrane Matrix, GFR (354230 / Corning Inc.)
(4) Collagen (1.6 $\mu$g/ml): collagen type IV from bovine lens (acid solubilized) (0.5 mg/ml) (ASC-4-104-01 / Nippi. inc.)

<Culture cells and their acquisition sources, ID number>

(1) Hela: Cell Resource Center for Biomedical Research, Institute of Development, Aging and Cancer, Tohoku University, TKG0331
(2) HepG2: Cell Resource Center for Biomedical Research, Institute of Development, Aging and Cancer, Tohoku University, TKG0205
(3) A549: Cell Resource Center for Biomedical Research, Institute of Development, Aging and Cancer, Tohoku University, TKG0184
(4) MCF7: Cell Resource Center for Biomedical Research, Institute of Development, Aging and Cancer, Tohoku University, TKG0479
(5) 293:: Riken BioResource Research Center, RCB1637
(6) iPS 409B2: Riken BioResource Research Center, HPS0076

<Culture condition of each cell>

(1) iPS cells

(1-1) Seeding density

28.5 colony/cm$^2$ (calculation based on 500 cells/colony) or $1.5 \times 10^5$ cells/cm$^2$ in terms of single cells.

(1-2) Culture liquid

A mixed liquid of (a):(b):(c):(d):(e) = 100 mL : 390 mL : 5 mL : 5 mL : 3.5 $\mu$l was used.
Note that (a) to (e) in the above represent:

(a) SSR (191-18375 / FUJIFILM Wako Pure Chemical Corp.)
(b) DMEM-F12 (046-32275 / FUJIFILM Wako Pure Chemical Corp.)
(c) 200 mM L-alanyl-L-glutamine (01102-82 / Nacalai Tesque Inc.)
(d) MEM Non-essential amino acid solution (16224004 / Nacalai Tesque Inc.)
(e) 2-Mercaptoethanol (135-07522 / FUJIFILM Wako Pure Chemical Corp.)

(1-3) Peeling liquid

A mixed liquid of (a):(b) = 0.25 g : 375 mL was used.

Note that (a) and (b) in the above represent:

(a) Disperse II (38302281 / FUJIFILM Wako Pure Chemical Corp.)
(b) DMEM-F12 (046-32275 / FUJIFILM Wako Pure Chemical Corp.)

(1-4) Procedure of culturing

A detailed procedure of culturing will be described below.

(1-4-1) Reagents

Dulbecco's modified eagle's medium (DMEM; Nissui Pharmaceutical Co., Ltd. 05919)
D-MEM/Ham's F-12 (DMEM-F12; FUJIFILM Wako Pure Chemical Corp. 04230795)
Stem Sure Replacement (SSR) (FUJIFILM Wako Pure Chemical Corp. 04632275)
Fetal bovine serum (FBS; BioWest)
MEM Non-essential amino acid solution (100×) (Nacalai Tesque Inc. 06344-56)
200 mM L-alanyl-L-glutamine (Powder; Nacalai Tesque Inc. 01102-82)
2-Mercaptoethanol (FUJIFILM Wako Pure Chemical Corp. 13706862)
Mitomycin C (FUJIFILM Wako Pure Chemical Corp. 139-18711)
Ethanol (Nacalai Tesque Inc. 14713-95)
Ethylene glycol (Nacalai Tesque Inc. 058-00986)
PBS (without Ca and Mg) (Nacalai Tesque Inc. 07269-84)
Gelatin, type B, powder, BioReagent suitable for cell culture (Sigma-Aldrich G9391-100G)
Trypsin powder (Nacalai Tesque Inc. 35547-64)
EDTA (Nacalai Tesque Inc. 15105-35)
Disperse II (FUJIFILM Wako Pure Chemical Corp. 38302281)
Dimethyl sulfoxide (DMSO; FUJIFILM Wako Pure Chemical Corp. 043-07216)
Acetamide (Nacalai Tesque Inc. 00117-32)
Propylene glycol (Nacalai Tesque Inc. 29218-35)
Bovin serum albumin (BSA; Nacalai Tesque Inc. 01281-84)
CHAPS (FUJIFILM Wako Pure Chemical Corp. 34104721)
Fibroblast growth factor (basic) (bFGF; PeproTech, Inc. AF-100-18B)
Y-27632 (Cayman 29218-35)
Cell Reservoir One (Nacalai Tesque Inc. 07485-44)

(1-4-2) Preparation of reagents

(1-4-2-1) L-glutamine solution

L-glutamine in an amount of 3.212 g is dissolved in ultrapure water to obtain a solution in an amount of 110 ml.

After dissolution, the solution is subjected to filtering sterilization using a 0.22 μm filter, aliquoted in 10ml fractions into 15ml centrifugation tubes, and stored at -20°C.

(1-4-2-2) 10% NaHCO$_3$ solution

NaHCO$_3$ in an amount of 10 g is dissolved in ultrapure water to obtain 100 ml of a 10% NaHCO$_3$ solution.

The solution is autoclaved for dissolution and sterilization and stored at room temperature.

(1-4-2-3) DMEM medium

Dulbecco's modified eagle's medium (DMEM) in an amount of 4.75 g is dissolved in ultrapure water to obtain a 500ml solution.

The solution is mixed using a stirrer for 30 minutes, autoclaved, and then stored.

(1-4-2-4) 0.1% gelatin solution

Gelatin in an amount of 0.5 g is dissolved in ultrapure water to obtain a 500ml solution.
The solution is autoclaved for dissolution and sterilization and stored at room temperature.

(1-4-2-5) MMC solution

A solution is prepared with the following formulation.

| | |
|---|---|
| Mitomycin C (MMC) | 10 mg |
| Ethanol | 1 ml |
| Ethylene glycol | 9 ml |

The mixture after dissolution is subjected to filtering sterilization using a 0.22μm filter to obtain a solution.

(1-4-2-6) Dispase solution (200 PU/ml)

A solution is prepared with the following formulation.

| | |
|---|---|
| Dispase II | 0.25 g |
| DMEM | 150 ml |

Upon dissolution, the solution is subjected to filtering sterilization using a 0.22μm filter, aliquoted in 1ml fractions into 15ml centrifugation tubes, and stored at -20°C (200 PU/ml).
When used, 9 ml of DMEM is further added to the solution for 10-fold dilution and the resulting solution is used.

(1-4-2-7) Medium for human iPS cell

A medium is prepared with the following formulation.

| | |
|---|---|
| DMEM-F12 | 390 ml |
| Non-essential amino acid | 5 ml |
| 200mM L-Glutamine | 5 ml |
| SSR (20%) | 100 ml |
| 2-Mercaptoethanol (0.1 mM) | 3.5 μl |

The prepared medium is stored at 4°C and used within two weeks.

(1-4-2-8) basic FGF solution

With the following formulation, the solvent is dissolved and subjected to filtering sterilization using a 0.22μm filter to prepare a solution.

| | |
|---|---|
| BSA | 0.05 g |
| CHAPS | 0.01 g |
| D-PBS (-) | 10 ml |

(1-4-2-9) Cryopreservation solution DAP213

According to the following procedure, 2M DMSO, 1M acetamide, 3M propylene glycol/human iPS cell medium (DAP213) is prepared.
Acetamide in an amount of 0.59 g is dissolved in about 6 ml of an iPS medium. The resulting solution is subjected to filtering sterilization using a 0.22μm filter and transferred to a 15ml centrifugation tube.
DMSO in an amount of 1.42 ml and propylene glycol in an amount of 2.2 ml are added to the above solution.
The medium is added to the solution to a volume of 10 ml. The resulting solution is mixed well, aliquoted in 0.5ml fractions into cryotubes, and stored at -80°C. Freezing and thawing can be repeated several times.

(1-4-3) Preparation of feeder cells

(1-4-3-1) Thawing of STO or mouse embryonic fibroblast (MEF)

1) Ten percent FBS/DMEM medium is brought back to room temperature.
2) Ten percent FBS/DMEM medium in an amount of 8 ml is transferred to a 15ml conical tube.
3) Cryopreserved cells are thawed in a 37°C water bath until just prior to the complete thawing.
4) The cells are transferred to the 15ml tube, which preliminarily contains the medium, by using a 1ml disposable pipette. The inside of the tube is rinsed with 1 ml of the fresh 10% FBS/DMEM medium to recover the remaining cells.
5) Centrifugation is performed at 1,500 rpm for 3 to 5 minutes.
6) After sucking off the supernatant, 10 ml of the 10% FBS/DMEM medium is added. Pipetting is sufficiently performed to suspend the cells until there is no cell aggregate.
7) The total cell suspension is transferred to a f100 dish.
8) The cells are cultured overnight at 37°C in a $CO_2$ incubator for adhesion and proliferation.
9) Next day, a state of the cells is observed under a microscope. The 10% FBS/DMEM medium is brought back to room temperature.
10) The medium in the dish is removed and 10 ml of the 10% FBS/DMEM medium is added to the dish.
11) The cells are cultured up to a confluent state at 37°C in the $CO_2$ incubator.

(1-4-3-2) Mitomycin C treatment

1) It is confirmed whether the cells reach a confluent state.
2) Mitomycin C is added to the f100 dish to a final concentration of 10 $\mu$g/ml (100 $\mu$l of a 1mg/ml mitomycin C stock solution is added to each 10 ml of the 10% FBS/DMEM medium). The dish is sufficiently swirled, so that the liquid is spread to the entire cells.
3) The cells are allowed to stand still at 37°C for 2 to 3 hours in the $CO_2$ incubator.
4) After 2 to 3 hours, the medium in the dish is removed and the cells are rinsed with 5 ml or more of PBS(-) three times.
5) The fresh 10% FBS/DMEM medium in an amount of 10 ml is added.
6) The cells are cultured overnight at 37°C in the $CO_2$ incubator.
7) Under this condition, most cells become non-dividing cells.
8) The cells are rinsed with 5 ml of PBS(-). After sucking off and discarding the PBS(-), 1 ml of 0.25% trypsin-EDTA is added to the cells and the cells are allowed to stand still at 37°C for 2.5 to 3 minutes.
9) Ten percent FBS/DMEM medium in an amount of 3 ml is added to recover the cells into a 15ml centrifugation tube by pipetting.
10) Centrifugation is performed at 1,500 rpm for 3 to 5 minutes.
11) After sucking off the supernatant, 900 $\mu$l of Cell Reservoir One is added to the cells. The cells are aliquoted in 200 $\mu$l fractions into 2ml cryotubes and cryopreserved (1.95 $\times$ $10^6$ cells/tube, enough cells to be seeded in f60 $\times$ 2 dishes after thawing).

(1-4-3-3) Production of feeder cells

1) One day before, a 0.1% gelatin solution is added to a dish (the adding amount of the gelatin solution corresponding to the size of the dish is as shown in Table 1 below).
2) The cells are allowed to stand still at 37°C for 30 minutes or more in the $CO_2$ incubator.
3) Ten percent FBS/DMEM medium is brought back to room temperature and 5 ml of the 10% FBS/DMEM medium is transferred to a 15ml conical tube.
4) The cell number is counted using a fraction of the cells.
5) The cryopreserved cells are thawed in the 37°C water bath until just prior to the complete thawing.
6) The cells are transferred to the 15ml tube, which preliminarily contains the medium, by using a 1ml disposable pipette. The inside of the tube is rinsed with 1 ml of the fresh 10% FBS/DMEM medium to recover the remaining cells.

7) Centrifugation is performed at 1,500rpm for 3 to 5 minutes.

8) After sucking off the supernatant, 1 ml of the 10% FBS/DMEM medium is added. Pipetting is sufficiently performed to suspend the cells until there is no cell aggregate.

9) The amount of the medium to be added is calculated so that a cell concentration is adjusted to 0.8 to $1.0 \times 10^6$ cells/ml.

10) The gelatin solution is removed, and the cell suspension of the calculated amount is seeded in the f60 dish.

11) The cells are incubated overnight at 37°C in the $CO_2$ incubator.

[Table 1]

| Dish size | Volume of gelatin solution |
|---|---|
| 24-Well | 200~300$\mu$l |
| 12-Well | 500$\mu$l |
| 6-Well/35mm | 1ml |
| 60mm | 2ml |
| 100mm | 4~5ml |

(1-4-3-4) Subculture method of human iPS cells

1) By the day before, the feeder cells are prepared. (The following reagent volume is for one 60mm cell culture dish.)

2) The medium for human iPS cell and the dispase solution are brought back to room temperature.

3) The dispase solution in an amount of 0.5 ml is added to the dish and the liquid is spread to the entire surface of the cells. Then, the cells are heated at 37°C for 3 minutes in the $CO_2$ incubator.

4) A state of the cells is observed under the microscope and the dispase solution is removed.

5) The cells are incubated at 37°C for 10 minutes. The cells are desirably in a state in which half or more of the colonies become small and compact from the peripheries and start to detach from the feeder cells.

6) The medium for human iPS cell in an amount of 1 ml is added to detach the cells from the dish. The colonies are crushed to an appropriate size by pipetting several times (10 times or less). Gilson P-1000 is suitably used (pipetting is performed so as to obtain the cell number of about 100 cells per colony of the iPS cells). When the colony is too small, the efficiency of re-adhesion and proliferation becomes extremely low. Thus, the colonies are detached while being monitored under the microscope.

7) Centrifugation is performed at 700 rpm for 2 minutes and the supernatant is removed as much as possible.

8) The medium is removed from the dish of the feeder cells and 3 ml of the medium for human iPS cell is added to each dish.

9) The medium in an amount of 1.5 to 2 ml is added to the conical tube to gently suspend the cells. The colonies of the iPS cells are already adjusted to the proper size of about 100 cells, and thus strong pipetting should be avoided. If a large colony is observed, the cell suspension is sucked by the pipette and a tip of the pipette is lightly pressed against the bottom of the conical tube. Then, the cell suspension is slowly discharged from the pipette to loosen the cells. The human iPS cell suspension in an amount of 1 ml is added to each feeder cell dish. bFGF is added to a final concentration of 15 ng/ml (if necessary, a Y-27532 solution is added to 10 $\mu$M/ml.)

10) A state of the cells is observed under the microscope. The dish is sufficiently swirled so that the colonies of the iPS cells are spread to the entire dish.

11) The cells were cultured overnight at 37°C in the $CO_2$ incubator.

12) Next day, a state of the cells is observed under the microscope and the medium is replaced.

13) Thereafter, the medium is replaced daily. The cells reach a confluent state in 3 to 4 days.

(1-4-3-5) Cryopreservation method of human iPS cells

1) One f60 dish containing the human iPS cells in the confluent state is prepared.

2) The medium for human iPS cell and the dispase solution are brought back to room temperature. Liquid nitrogen and ice are prepared.

A cryopreservation tube (Nulgene #5000-1012) is labeled with "cell name," "date," "passage number," and the like and cooled on the ice.

3) The cryopreservation solution DAP213 is thawed and cooled on the ice.

4) The medium of the dish of the human iPS cells is removed and an appropriate amount of PBS(-) is added for rinsing and then discarded.

5) The dispase solution in an amount of 500 $\mu$l is added to the dish and the liquid is spread to the entire surface of the cells. Then, the cells are heated at 37°C for 3 minutes in the $CO_2$ incubator.

6) A state of the cells is observed under the microscope and the dispase solution is removed.

7) The cells are incubated at 37°C for 10 minutes.

8) The medium for human iPS cell in an amount of 3 ml is added to detach the entire cells from the dish.

9) The cells are recovered in a 15ml centrifugation tube.

10) Centrifugation is performed at 1,500 rpm for 3 to 5 minutes and the supernatant is removed as much as possible.

11) DAP213 in an amount of 200 $\mu$l is added and the cells are gently suspended. The cells are transferred to the cryopreservation tube prepared in advance. The cryotube is held by tweezers and submerged in liquid nitrogen. Due to high cytotoxicity of DAP, this operation should be performed as quickly as possible. As a general guide, 15 seconds or less is desirable. Further, the addition amount of DAP may be 200 $\mu$l regardless of the number of the cells to be frozen.

12) The cells are frozen in liquid nitrogen for 30 seconds to 1 minute, so that the cells are completely frozen to the inside.

13) The tube is moved to a liquid nitrogen storage container.

(1-4-3-6) Thawing of human iPS cells

1) By the day before, the feeder cells corresponding to one f60 dish are prepared for each cryotube of the human iPS cells.

2) The medium for human iPS cell in an amount of 10 ml is transferred to a 15ml conical tube and heated in the 37°C water bath.

3) The medium for human iPS cell in an amount of 1 ml heated to 37°C in advance is added to the cryotube of the human iPS cells which have been cryopreserved, and the cells are rapidly thawed by pipetting. Thawing and dilution should be performed quickly. Further, thawing in a water bath causes a significant reduction in a cell survival rate after thawing.

4) After repeating the above operations several times, the cell suspension is recovered in a 15ml centrifugation tube, and centrifugation is performed at 1,500 rpm for 3 to 5 minutes.

5) The supernatant is sucked off and discarded, and 1 ml of the medium for human iPS cell is added. Pipetting is gently performed to suspend the cells to such an extent that the colonies of the iPS cells do not become too small. In order to loosen the large colonies, the cell suspension is sucked by the pipette and a tip of the pipette is lightly pressed against the bottom of the centrifugation tube, and then the cell suspension is slowly discharged from the pipette.

6) The medium for the feeder cell is sucked off and removed and 3 ml of the medium for human iPS cell is added.

7) The total amount of the cells is seeded and the bFGF solution is added to 15 ng/ml (if necessary, the Y-27532 solution is added to 10 $\mu$M/ml).

8) A state of the cells is confirmed under the microscope.

9) The cells are cultured overnight at 37°C in the $CO_2$ incubator.

10) Next day, a state of the cells is confirmed under the microscope. In general, many cells are confirmed to be dead the next day after thawing. The medium is continually replaced once a day. In general, the cells become ready to be subcultured in about 3 days.

(2) Cells other than iPS cells

(2-1) Seeding density

$$7.8 \times 10^4 \text{ cells/cm}^2$$

(2-2) Culture liquid

A mixed liquid of (a):(b) = 50 : 450 (mL) was used.
Note that, in the above, (a) and (b) respectively represent:

(a) FBS (Biowest)
(b) Dulbecco's modified eagle's medium (DMEM; Nissui Pharmaceutical Co., Ltd. 05919)

(2-3) Peeling solution

A mixed liquid of (a):(b):(c) = 1.25 g : 0.2 g : 500 mL was used.
Note that, in the above, (a) to (c) respectively represent:

(a) trypsin (35547-64 / Nacalai Tesque Inc.)
(b) EDTA (15105-35 / Nacalai Tesque Inc.)
(c) PBS(-) (07269-84 / Nacalai Tesque Inc.)

(2-4) Procedure for culturing

A detailed procedure for culturing will be described below.

(2-4-1) DMEM medium

Dulbecco's modified eagle's medium (DMEM; Nissui Pharmaceutical Co., Ltd. 05919): 433 ml (4.75 g is dissolved in ultrapure water and autoclaved)
A 10%w/w $NaHCO_3$ (Nacalai Tesque Inc.) solution: 7 ml (0.7 g is dissolved in ultrapure water and autoclaved)
A 200mM L-glutamine (Nacalai Tesque Inc.) solution: 10 ml (0.292 g is dissolved in ultrapure water and filtrated)
FBS (Biowest): 50 ml (inactivated)

(2-4-2) Trypsin/EDTA solution

Trypsin (Nacalai Tesque Inc.): 1.25 g, EDTA (Nacalai Tesque Inc.): 0.2 g, PBS(-): 500 ml
The dissolved solution is filtrated, aliquoted in 10ml fractions, and stored at -20°C.

(2-4-3) Culturing/subculturing

1) The frozen cell line is thawed at 37°C in a water bath (500 µl of frozen liquid)
2) The cells are recovered in 5 ml of the DMEM medium, that is, 10 times the volume of the cells, followed by centrifugation at 1,500 rpm for 3 minutes.
3) The supernatant is discarded, and the cells are seeded in an f60 or f100 dish.
4) The cells are cultured in an incubator for several days.
5) Depending on the cells to be cultured, subculturing is performed as follows (Hela, A549, HepG2, UV♀2, MCF7: 2 to 3 days, Hek293: 4 to 7 days).

• Cells other than Hek293

1) The medium is sucked off and discarded. An appropriate amount of PBS(-) is added and then PBS(-) is sucked and discarded.
2) The trypsin/EDTA solution is added to f60 in an amount of 500 µl and to f100 in an amount of 1 ml, followed by incubation at 37°C for 2.5 to 3 minutes.
3) The DMEM medium in an amount two or more times greater than that of the trypsin/EDTA solution is added to recover the cells. Several tens of µl of the cells are used to count the cell number and the remaining cells are subjected to centrifugation at 1,500 rpm for 3 minutes.
4) The cells are seeded in a 4-well dish at $3.0 \times 10^5$ cells/well.

5) The cells are cultured in the incubator. The medium is replaced every other day.

• Hek293

1) The medium is sucked off and discarded, and an appropriate amount of PBS(-) is added.
2) The cells are recovered by pipetting and several tens of $\mu$l of the cells are used to count the cell number. The remaining cells are subjected to centrifugation at 1,500 rpm for 3 minutes.
3) The cells are seeded in a 4-well dish at $3.0 \times 10^5$ cells/well.
4) The cells are cultured in the incubator. The medium is replaced every other day.

[Production of micro-stamp]

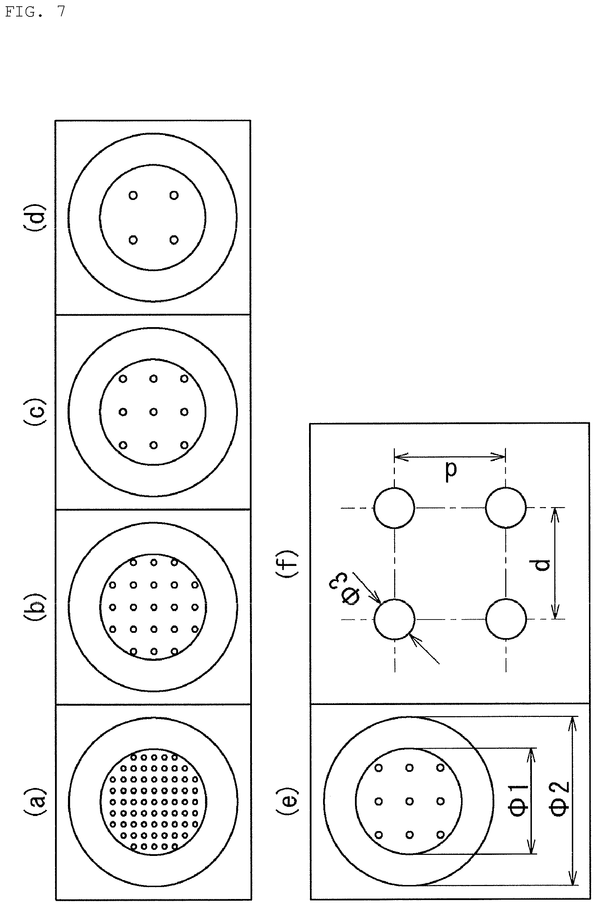

A production method of the micro-stamp used in each Example will be described.
First, as a photomask, 4 kinds of photomasks shown in Figs. 7(a) to (d) were used to produce 4 kinds of molds.
The size of each photomask is summarized in Table below. The meaning of $f_1$ to $f_3$ and d is the same as shown in Figs. 7(e) and (f). $f_1$ represents a diameter of the inner circle of the mask, $f_2$ represents a diameter of the outer circle of the mask, $f_3$ represents a diameter of each dot in the mask, and d represents a distance between center points of two adjacent dots.

[Table 2]

| | Photomask 1 | Photomask 2 | Photomask 3 | Photomask 4 |
|---|---|---|---|---|
| | Fig. 5 (a) | Fig. 5 (b) | Fig. 5 (c) | Fig. 5 (d) |
| $\phi$ 1 (mm) | 16 | | | |
| $\phi$ 2 (mm) | 10 | | | |
| $\phi$ 3 ($\mu$m) | 250 | 500 | 750 | 1000 |
| d (mm) | 1.00 | 2.00 | 3.00 | 4.00 |

In a specific procedure for production of the mold, as shown in Fig. 1, a resist is applied onto a silicon substrate, the resist is subjected to heat curing using a heater, and then the resist is exposed to light through each photomask described above, followed by developing and cleaning, to produce the mold.
Next, 4 kinds of the molds produced in the above-mentioned procedure were used to produce 4 kinds of the micro-stamps.
As a specific procedure, in accordance with the above-mentioned procedure shown in Fig. 2, the mold is subjected to a release treatment using a release agent and then polydimethylsiloxane (PDMS) is poured in the mold and dried using a constant-temperature dryer to produce the micro-stamp.
Hereinafter, the micro-stamps corresponding to the photomasks 1 to 4 in the above-mentioned Table 2 are referred to as micro-stamps 1 to 4, respectively.

[Verification test of effects of invention]

Various verification tests were performed as follows using the cell adhesion molecules and the cells prepared in the above-mentioned procedures. In some Examples, the micro-stamps prepared in the above-mentioned procedures were used.

<Example 1>

A fluororesin (CYTOP CTX-809A, manufactured by AGC Inc.) was applied on a planar quartz substrate by spin coating. After spin coating, the substrate was placed on a heater and baked at 200°C for one hour.
A micro-stamp 1 ($f_3$ = 250 $\mu$m) was used to form a pattern of Matrigel, which was used as a cell adhesion molecule, on the fluororesin layer in accordance with the above-mentioned procedure shown in Fig. 3.
Next, in accordance with the above-mentioned procedure shown in Fig. 6, cell culturing and recovery of spheroids were performed.

That is, the cell culture scaffold thus produced was covered with the culture liquid and the cells were seeded. As the cells, the iPS cells prepared as described above were used, and the cells were cultured in DMEM + 10% FBS as a medium under the conditions of 37°C and a $CO_2$ concentration of 5%.

The number of the cells to be seeded was set to $1.6 \times 10^5$ cells/cm$^2$, and the culture period was set to 10 days. After 10 days, the cells were detached from the scaffold by applying extremely gentle stimulation by pipetting and the detached cells were observed under a phase contrast microscope. The observation was performed every day or every other day, and the medium was replaced every other day.

<Example 2>

The cell culture scaffold was produced in the same manner as in Example 1 except that a micro-stamp 2 ($f_3$ = 500 $\mu$m) was used instead of the micro-stamp 1, and the cells were cultured.

<Example 3>

The cell culture scaffold was produced in the same manner as in Example 1 except that a micro-stamp 3 ($f_3$ = 750 $\mu$m) was used instead of the micro-stamp 1, and the cells were cultured.

<Example 4>

The cell culture scaffold was produced in the same manner as in Example 1 except that a micro-stamp 4 ($f_3$ = 1,000 $\mu$m) was used instead of the micro-stamp 1, and the cells were cultured.

<Examples 5 to 20>

Furthermore, tests were performed with various combinations of the culture cells and the cell adhesion molecules as shown in Table 3 below in accordance with the method in Example 1.

Note that Examples related to the iPS cells (Examples 8 and 17) used cell culture scaffolds in which the various cell adhesion molecules were formed in a pattern on the fluororesin layers using the micro-stamp 2 ($f_3$ = 500 $\mu$m) or the micro-stamp 3 ($f_3$ = 750 $\mu$m). On the other hand, for other cells, the cell culture scaffolds to be used were produced by forming the various cell adhesion molecules in a dot-like shape (a diameter of about 1,000 $\mu$m for fibronectin and vitronectin and a diameter of about 1,500 $\mu$m for fibronectin and vitronectin) on the fluororesin layers using pipettes.

[Table 3]

| | Substrate | Type | Cell adhesion molecule | Initial adhesiveness pattern formation | Spheroid |
|---|---|---|---|---|---|
| Example 5 | (HEK)293 | Mesenchymal cell | Matrigel | ◯ | △ |
| Example 6 | Hela | Fibroblast-like cell | Matrigel | ◯ | △ |
| Example 7 | HepG2 | Epithelial-like cell | Matrigel | ◯ | ◯ |
| Example 8 | iPS | Epithelial-like cell | Matrigel | ◯ | ◯ |
| Example 9 | MCF7 | Epithelial-like cell | Matrigel | ◯ | ◯ |
| Example 10 | (HEK)293 | Mesenchymal cell | Vitronectin | ◯ | △ |
| Example 11 | Hela | Fibroblast-like cell | Vitronectin | ◯ | △ |
| Example 12 | HepG2 | Epithelial-like cell | Vitronectin | ◯ | ◯ |
| Example 13 | MCF7 | Epithelial-like cell | Vitronectin | ◯ | ◯ |
| Example 14 | (HEK)293 | Mesenchymal cell | Fibronectin | ◯ | △ |
| Example 15 | Hela | Fibroblast-like cell | Fibronectin | ◯ | △ |
| Example 16 | HepG2 | Epithelial-like cell | Fibronectin | ◯ | ◯ |
| Example 17 | iPS | Epithelial-like cell | Fibronectin | ◯ | ◯ |
| Example 18 | MCF7 | Epithelial-like cell | Fibronectin | ◯ | ◯ |

(continued)

|  | Substrate | Type | Cell adhesion molecule | Initial adhesiveness pattern formation | Spheroid |
|---|---|---|---|---|---|
| Example 19 | A549 | Squamous cell | Collagen | ○ | △ |
| Example 20 | MCF7 | Epithelial-like cell | Collagen | ○ | △ |

[Verification test results]

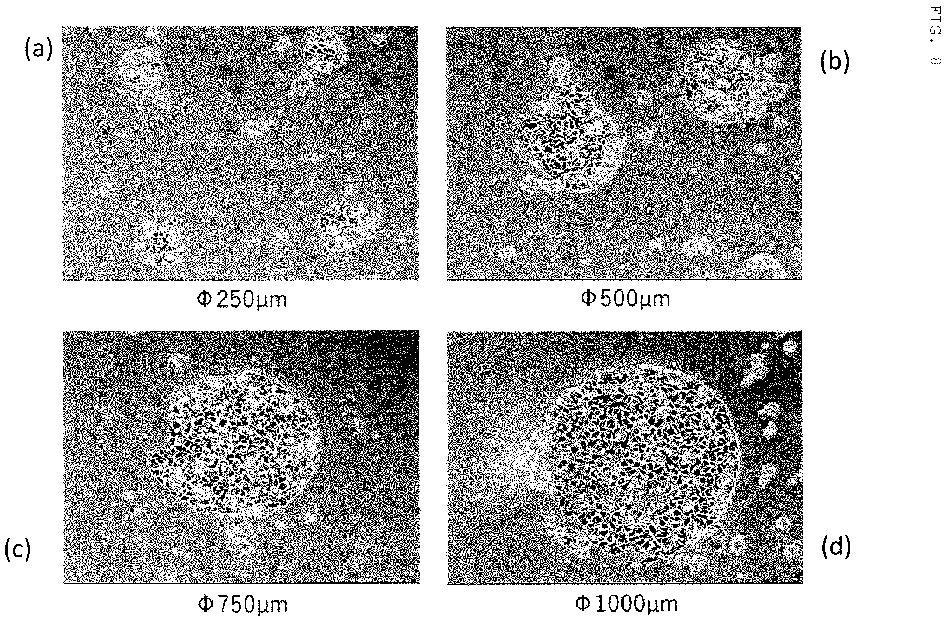

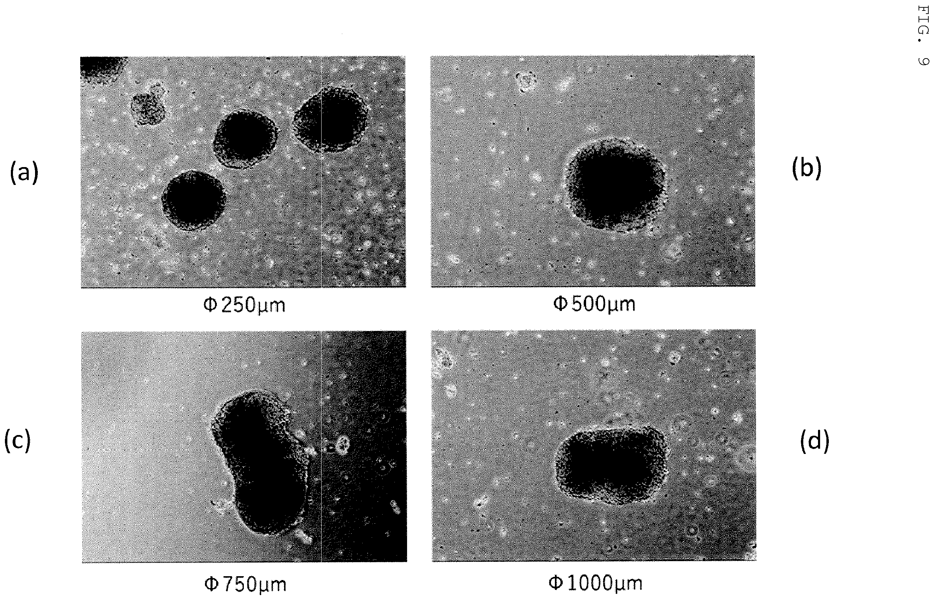

Fig. 8 shows photographs of the cell culture products before detachment in Examples 1 to 4. Fig. 8(a), Fig. 8(b), Fig. 8(c), and Fig. 8(d) show photographs of the cell culture products before detachment in Examples 1, 2, 3, and 4, respectively. Further, Fig. 9 shows photographs of the cell culture products after detachment. Fig. 9(a), Fig. 9(b), Fig. 9(c), and Fig. 9(d) show photographs of the cell culture products after detachment in Examples 1, 2, 3, and 4, respectively.

As seen in Fig. 8 and Fig. 9, it was found that, in all Examples, the cell culture products (spheroids) were formed, and could be detached in the culture liquid without being destroyed and then recovered.

Furthermore, it was found that the size of the spheroids can be controlled according to the dimension of the dots.

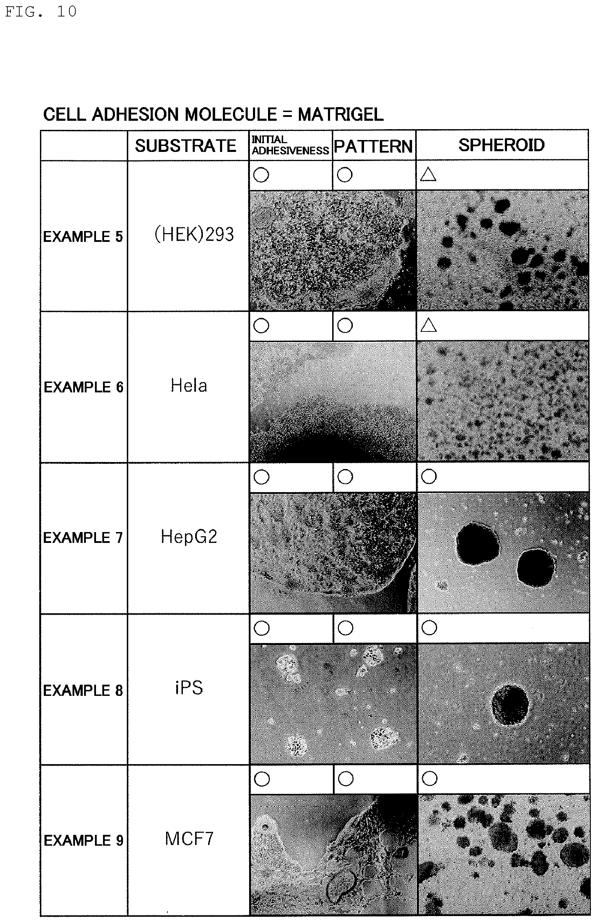

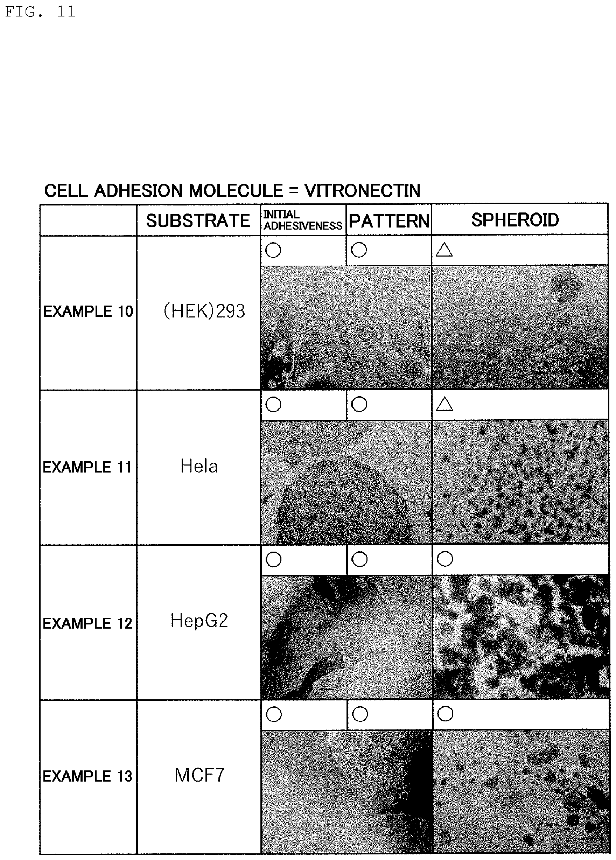

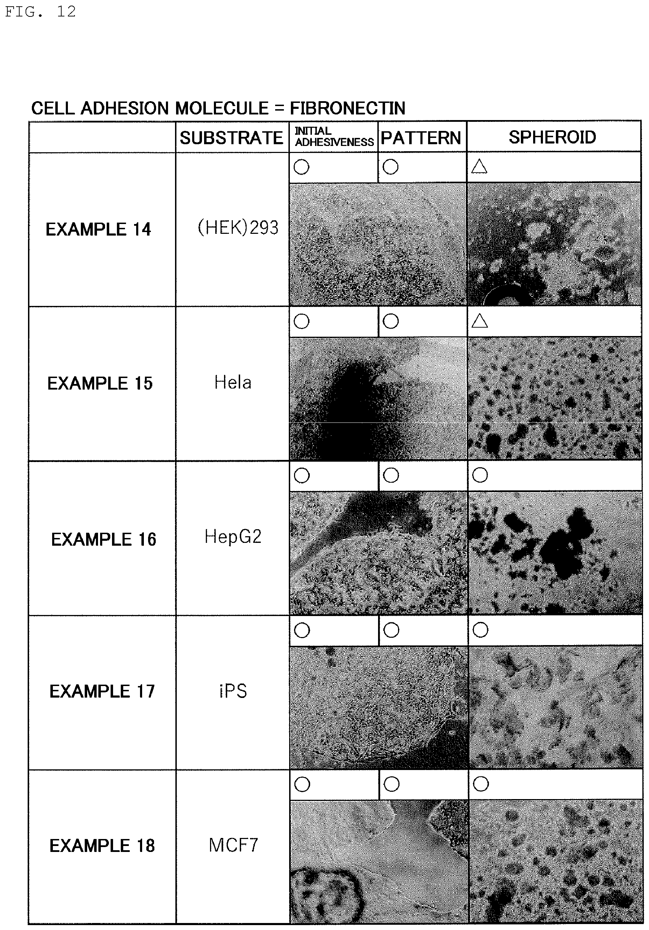

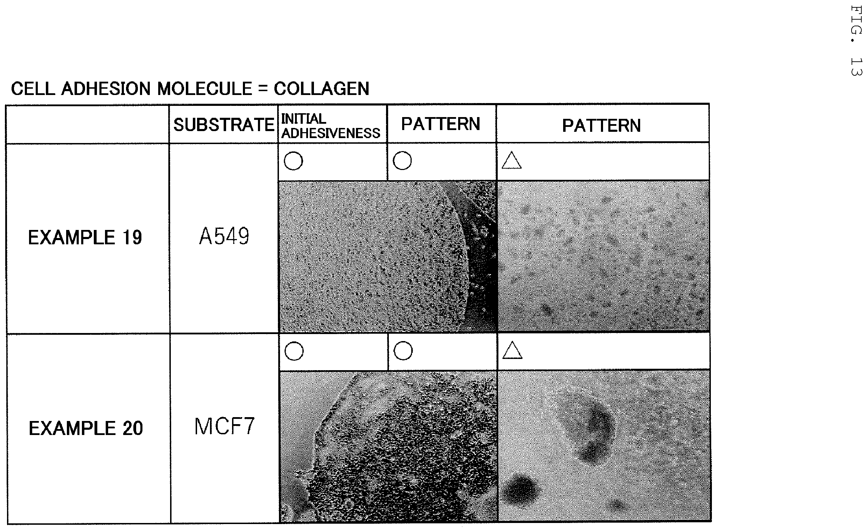

Fig. 10 to Fig. 13 show photographs obtained in Examples 5 to 20. Initial adhesiveness/pattern formability and spheroid formation were evaluated, and these results were shown in the above-described Table 3 as well as in Fig. 10 to Fig. 13.

Note that spheroid formation was evaluated according to the following criteria.

Circle symbol: cell aggregates are sufficiently thick and large in three dimensions and can be recognized as spherical in shape.

Triangle symbol: cell aggregates are fairly thick and large in three dimensions.

Cross symbol: cells are not aggregated for the most part and are two-dimensionally dispersed.

(Thickness of cells reduces transmitted light during microscopic observation and cells appear to be dark in color.)

As shown in Table 3 and Fig. 10 to Fig. 13, it was found that, in the various combinations of the culture cells and the cell adhesion molecules, the cell culture products (spheroids) were formed, and could be detached into the culture liquid without being destroyed and then recovered.

[Sealing method]

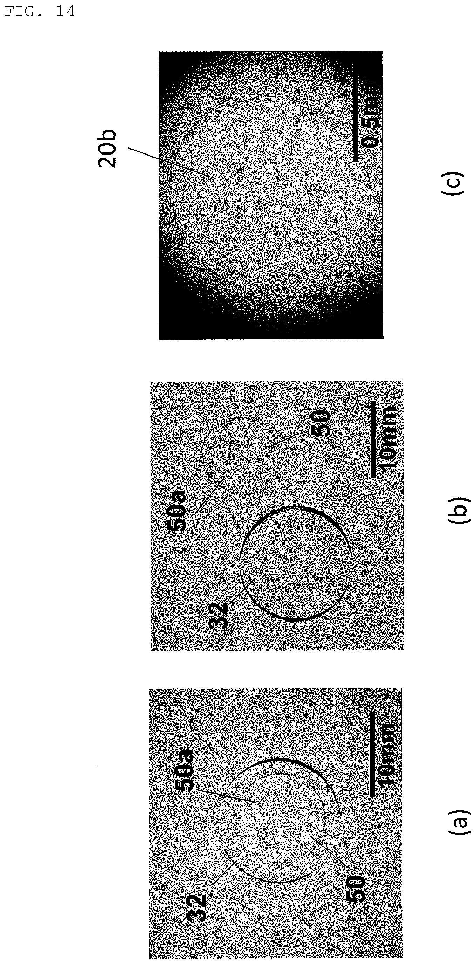

Fig. 14 shows an example of the pattern of the cell adhesion molecules 20b produced by the sealing method. The substrate 32 in use was obtained by applying a fluororesin (CYTOP CTX-809A, manufactured by AGC Inc.) to a planar quartz substrate by spin coating. The sealing material 50 was formed by applying an ultraviolet curable resin (manufactured by Dymax) to the substrate 32 by spin coating, performing ultraviolet-curing using a negative mold with a dot pattern as a mask, and then removing uncured parts. In Fig. 14(a), the sealing material 50 in which 4 holes 50a are formed is formed on the substrate 32. A scale bar indicates 10 mm.

Fig. 14(b) shows a state in which the sealing material 50 is peeled off from the substate 32. When the sealing material 50 was peeled off from the substate 32, there was no component of the sealing material 50 left on the substate 32. Thus, the sealing material 50 could be cleanly peeled off.

Fig. 14(c) is a photograph of the patterned cell adhesion molecules 20b (cell adhesion molecule regions) which were obtained by applying the cell adhesion molecules 20b to the sealing material 50 in a state of being formed on the substate 32 and peeling off the sealing material 50 after one-hour fixation. The cell adhesion molecules 20b with a cylindrical shape having a diameter of 1 mm and thickness of 0.1 mm could be obtained. The cell adhesion molecules 20b on the substate 32 serve as the cell culture scaffold according to the present invention. Further, a product in which the sealing material 50 in which the holes 50a are formed is closely disposed on the substate 32 in Fig. 14(a) serves as the cell culture kit for forming spheroids according to the present invention.

Next, the spheroids were produced by using the cell culture scaffold produced by the sealing method.

First, the substrate 32 attached with the sealing material 50 in which the holes 50a having a diameter of 1 mm (= 1,000 μm) were formed, shown in Fig. 14, was subjected to gas sterilization.

Next, Matrigel was added from the top of the sealing material 50 (disposing the cell adhesion molecules) and fixed at 37°C for 1 hour in an incubator.

The sealing material 50 was peeled off by using tweezers, which had been subjected to flame sterilization, to obtain the cell culture scaffold in which the cell adhesion molecule (Matrigel) dots having a diameter of 1,000 μm were

formed in a pattern.

Next, the cells (Hek293) used in Examples 5, 10, and 14 were seeded on the substrate 32. Then, the cells were cultured by the same culture method as in the Example described above (the method described in "(2-4) Procedure for culturing").

After seeded, the cells were detached from the substrate 32 by pipetting on Day 5 and observed on Day 6.

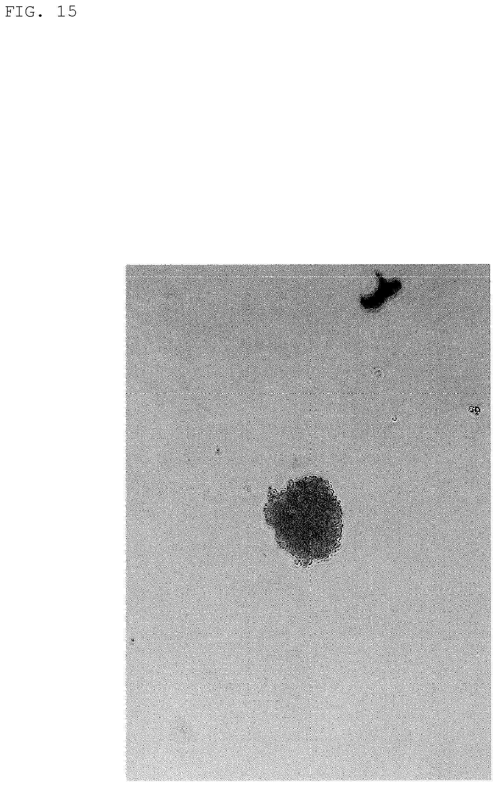

Fig. 15 shows an observation result. The spheroids of the cells Hek293 could be obtained.

Industrial Applicability

The cell culture scaffold according to the present invention can be suitably used to obtain a cell culture product in a spheroid-like state.

Reference Signs List

| | |
|------|------|
| 9 | outer container |
| 10 | mold |
| 11 | substrate |
| 12 | resist |
| 13 | heater |
| 14 | light source |
| 15 | photomask |
| 20 | micro-stamp |
| 20b | cell adhesion molecule |
| 20bh | thickness |
| 21 | rubber tube |
| 22 | release agent |
| 23 | stamp material |
| 24 | constant-temperature dryer |
| 20a | projection portion |
| 32 | substrate |
| 32a | (water-repellent) surface |
| 41 | culture liquid |
| 42 | culture product |
| 50 | sealing material |
| 50a | hole |

**Claims**

1. A cell culture scaffold comprising: a substrate having a water-repellent surface made of a fluororesin; and a cell adhesion molecule formed on the water-repellent surface of the substrate.

2. The cell culture scaffold according to claim 1, wherein the cell adhesion molecule is an extracellular matrix.

3. The cell culture scaffold according to claim 1 or 2, wherein the cell adhesion molecule is formed in a pattern on the water-repellent surface of the substrate.

4. The cell culture scaffold according to any one of claims 1 to 3, wherein the cell adhesion molecule is formed in a shape of a dot with a diameter of 100 to 1,000 $\mu$m.

5. A method for producing a cell culture product, comprising a step of culturing, using the cell culture scaffold according to any one of claims 1 to 4, cells on a surface of the cell adhesion molecule.

6. The method for producing a cell culture product according to claim 5, comprising a step of, by utilizing a reduction in adhesive force at a boundary face between the water-repellent surface of the substrate and the cell adhesion molecule after a predetermined period of time from a start of cell culturing, detaching the cell adhesion molecule spontaneously or by applying stimulation to the boundary face and then recovering the cell adhesion molecule.

7.  The method for producing a cell culture product according to claim 5 or 6, wherein the cell is an adherent cell.

8.  A method for producing a cell culture scaffold comprising:

a step of disposing a sealing material, in which one or more holes are formed, on a substrate having a water-repellent surface made of a fluororesin;
a step of disposing a cell adhesion molecule on a top of the sealing material; and
a step of peeling off the sealing material.

9.  A cell culture kit for forming spheroids, wherein a sealing material, in which one or more holes are formed, is closely disposed on a substate having a water-repellent surface made of a fluororesin.

10. The cell culture kit for forming spheroids according to claim 9, wherein a plurality of the holes different in size are formed.

FIG. 1

FIG. 2

FIG. 3

FIG. 4

FIG. 5

FIG. 6

(a)

(b)

(c)

FIG. 7

FIG. 8

(a)  Φ250μm

(b)  Φ500μm

(c)  Φ750μm

(d)  Φ1000μm

FIG. 9

FIG. 10

CELL ADHESION MOLECULE = MATRIGEL

| | SUBSTRATE | INITIAL ADHESIVENESS | PATTERN | SPHEROID |
|---|---|---|---|---|
| EXAMPLE 5 | (HEK)293 | ◯ | ◯ | △ |
| EXAMPLE 6 | Hela | ◯ | ◯ | △ |
| EXAMPLE 7 | HepG2 | ◯ | ◯ | ◯ |
| EXAMPLE 8 | iPS | ◯ | ◯ | ◯ |
| EXAMPLE 9 | MCF7 | ◯ | ◯ | ◯ |

FIG. 11

CELL ADHESION MOLECULE = VITRONECTIN

| | SUBSTRATE | INITIAL ADHESIVENESS | PATTERN | SPHEROID |
|---|---|---|---|---|
| EXAMPLE 10 | (HEK)293 | ○ | ○ | △ |
| EXAMPLE 11 | Hela | ○ | ○ | △ |
| EXAMPLE 12 | HepG2 | ○ | ○ | ○ |
| EXAMPLE 13 | MCF7 | ○ | ○ | ○ |

FIG. 12

CELL ADHESION MOLECULE = FIBRONECTIN

| | SUBSTRATE | INITIAL ADHESIVENESS | PATTERN | SPHEROID |
|---|---|---|---|---|
| EXAMPLE 14 | (HEK)293 | ○ | ○ | △ |
| EXAMPLE 15 | Hela | ○ | ○ | △ |
| EXAMPLE 16 | HepG2 | ○ | ○ | ○ |
| EXAMPLE 17 | iPS | ○ | ○ | ○ |
| EXAMPLE 18 | MCF7 | ○ | ○ | ○ |

FIG. 13

EP 3 995 566 A1

CELL ADHESION MOLECULE = COLLAGEN

| | SUBSTRATE | INITIAL ADHESIVENESS | PATTERN | PATTERN |
|---|---|---|---|---|
| EXAMPLE 19 | A549 | ○ | ○ | △ |
| | | | | |
| EXAMPLE 20 | MCF7 | ○ | ○ | △ |
| | | | | |

FIG. 14

(a)

(b)

(c)

FIG. 15

## INTERNATIONAL SEARCH REPORT

International application No.

PCT/JP2020/026399

**A. CLASSIFICATION OF SUBJECT MATTER**

C12M 3/00(2006.01)i; C12N 5/02(2006.01)i
FI: C12M3/00 A; C12N5/02

According to International Patent Classification (IPC) or to both national classification and IPC

**B. FIELDS SEARCHED**

Minimum documentation searched (classification system followed by classification symbols)
C12M3/00; C12N5/02

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | US 5108926 A (BOARD OF REGENTS, THE UNIVERSITY OF TEXAS SYSTEM) 28.04.1992 (1992-04-28) abstract, claim 6, column 3, lines 1-14, column 4, lines 5-11, column 5, lines 36-58 | 1-3,5,7,8<br>1-5,7-10 |
| X<br>Y | 楠正暢 他, Polytetrafluoroethylene と Poly-D-Lysine を用いた神経細胞パターン作製法の検討 ("Nerve cell patterning using polytetrafluoroethylene and poly-D-lysine"), 電気学会論文誌 C, 2009, vol. 129, no. 11, pp. 8, 2014-2018, extended summary, abstract, p. 2015, right column, fig. 4, 5, p. 2017, column "summary", (KUSUNOKI, Masanobu et al., The transactions of the Institute of Electrical Engineers of Japan C) | 1,3,5,7<br>1-5,7-10 |
| Y | WO 2014/112633 A1 (TOYO GOSEI CO., LTD.) 24.07.2014 (2014-07-24) abstract, claims 1-19, paragraphs [0037]-[0041] | 1-5,7-10 |

☒ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 08 September 2020 (08.09.2020) | 15 September 2020 (15.09.2020) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

| International application No. |
|---|
| PCT/JP2020/026399 |

C (Continuation).   DOCUMENTS CONSIDERED TO BE RELEVANT

| Category* | Citation of document, with indication, where appropriate, of the relevant passages | Relevant to claim No. |
|---|---|---|
| A | 楠正暢 他，毛細血管作製を目的としたポリテトラフルオロエチレン基板上へのマイクロパターニング法の研究 ("Micro-patterning technique for capillary blood vessels on Polytetrafluoroethylene substrate"), Memoirs of the Faculty of Biology-Orientecl Science and Technology of Kinki University, 2010, vol. 25, pp. 1-5, abstract, fig. 1-4, summary (KUSUNOKI, Masanobu et al.) | 1-10 |

Form PCT/ISA/210 (continuation of second sheet) (January 2015)

| Patent Documents referred in the Report | Publication Date | Patent Family | Publication Date |
|---|---|---|---|
| US 5108926 A | 28 Apr. 1992 | (Family: none) | |
| WO 2014/112633 A1 | 24 Jul. 2014 | (Family: none) | |

**INTERNATIONAL SEARCH REPORT**
Information on patent family members

International application No.

PCT/JP2020/026399

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013099282 A

- JP 2016214210 A

**Non-patent literature cited in the description**

- **TAMURA.** Studies on apatite dot production methods for obtaining cell/spheroid arrays. *Japanese Society for Medical and Biological Engineering Symposium 2013,* 20 September 2013, 141

- Aging and Cancer. Tohoku University